# EUROPEAN PATENT SPECIFICATION

(11) **EP 3 453 381 B1**
(45) Date of publication and mention of the grant of the patent: **18.11.2020**
(21) Application number: 17190217.4
(22) Date of filing: 08.09.2017
(51) Int. Cl.: A61K 8/97, A61K 47/18, A61K 31/4188, A61K 31/197, A23L 33/105, A23L 33/15, A23L 33/175, A61K 9/48

(54) **METHOD AND USE OF A COMPOSITION FOR IMPROVING HAIR APPEARANCE**
VERFAHREN UND VERWENDUNG EINER ZUSAMMENSETZUNG ZUR VERBESSERUNG DES ERSCHEINUNGSBILDS DER HAARE
PROCÉDÉ ET UTILISATION D'UNE COMPOSITION PERMETTANT D'AMÉLIORER L'APPARENCE DES CHEVEUX

(43) Date of publication of application: 13.03.2019
(73) Proprietor: Bayer Consumer Care AG, 4052 Basel (CH)
(72) Inventor: Boudon, Stephanie, 4303 Kaiseraugst (CH); Kurka, Peter, 4002 Basel (CH); Gnacke, Christina, 50733 Köln (DE); Zhang, Lei, 8005 Zürich (CH); Meza, Sonia Margarita, 52774 Huixquilucan (MX)
(74) Representative: van der Vlugt, Stephan Michael

(56) References cited:
- WO-A1-95/05146
- ES-A1- 2 052 450
- Anonymous: "Hair Renew Formula (TM) Nourishes Thinning Hair* FEATURED PRODUCT 60 Softgels Hair Renew Formula Nourishes Thinning Hair*", , 30 April 2016 (2016-04-30), pages 1-2, XP055424096, Retrieved from the Internet: URL:http://www.euromedicausa.com/uploads/p roduct-literature-files/94-Patient-One-She et.pdf [retrieved on 2017-11-13]
- Anonymous: "Beipackzettel Priorin Kapseln", , 31 January 2011 (2011-01-31), pages 1-2, XP055424062, Retrieved from the Internet: URL:https://priorin.de/static/media/pdf/Pr iorin-Beipackzettel.pdf [retrieved on 2017-11-13]
- D. Rodney Sinclair: "Healthy Hair: What Is it?", Journal of Investigative Dermatology Symposium Proceedings, vol. 12, no. 2, 1 December 2007 (2007-12-01), pages 2-5, XP055632355, DOI: https://doi.org/10.1038/sj.jidsymp.5650046

## Description

The invention relates to the use of a composition for improved hair appearance and a method for obtaining shiny hair.

Hair loss, also known as *alopecia* or baldness, refers to a loss of hair from part of the head or body. Typically at least the head is involved. The severity of hair loss can vary from a small area to the entire body, although hair-loss on the head is of particular relevance. The reasons for hair-loss are diverse and may have external origin like e.g. traumata or drugs or may be the consequence of a disease like *Alopecia areata*, also known as spot baldness, which is an autoimmune disorder, or fungal infections such as *tinea capitis.* Beside these reasons even more relevant for hair-loss is androgenic hair-loss under which e.g. approximately 15 million men and/or women in Germany suffer and which results from the hormone dihydrotestosterone.

Compositions for improving hair growth and/or for compensating hair-loss are known in the art e.g. in the form of hair elixirs, shampoos, liquids, pharmaceuticals or food supplements. All these products mainly focus exclusively on regaining hair and/or minimizing hair-loss resulting in constant or larger hair volume.

As an example the Spanish national patent ES 2 052 450 A1 discloses a hair lotion comprising biotin,
L-cystine, millet extract and pantothenic acid and/or a salt thereof for use in controlling alopecia, stimulating hair growth and getting rid of grey hair, dandruff and seborrhea.

As a further example the featured product *Hair Renew Formula™* (URL:http://www.euromedicausa.com/ uploads/product-literature-files/94-Patient-One-Sheet.pdf) discloses a dietary supplement for nourishing thinning hair and sustaining healthy hair growth.

In another example the international patent application WO 95/05146 A1 discloses hair loss compositions of the digestive administration type, preferably for oral administration, characterized in that they are comprised of, in effective amounts, one and preferably a plurality of constituants like inositol; dry sesame extract and/or millet extract; spirulina or "issegue" algae; zinc; magnesium; with preferably the following constituents; sulphured amino-acids like L-cystine; vitamins like vitamin H (biotin) and/or vitamin B₅ (pantothenic acid); trace minerals.

However, none of these products specifically addresses other parameters of existing or regained hair which are also important for a generally good and healthy appearance of the hair. Thus, there is a need for easy applicable compositions that can be used to support the good and healthy appearance of hair, and methods for obtaining hair having a healthy and good appearance and at the same time that these compositions also support to regain hair and/or minimize hair-loss.

It was surprisingly found that the use of the below compositions significantly improve the appearance of hair after applying the food supplement for at least eight weeks and at the same time generate hair having a larger volume. Thus, a first aspect of the present invention relates to the use of a food supplement in the form of a capsule, wherein the food supplement comprises
a. biotin in an amount of 0,05 to 2 mg based on each capsule, and
b. L-cystine in an amount of 1 to 5 mg based on each capsule, and
c. millet extract in an amount of 100 to 500 mg based on each capsule, and
d. pantothenic acid and/or a salt thereof in an amount of 5 to 50 mg based on each capsule, and
e. optionally at least one auxiliary ingredient
for obtaining shiny hair or soft and elastic hair or smooth hair moisturized hair or vital hair when applied in the form of two capsules once a day on a daily basis for at least eight weeks.

The second aspect of the invention is the non-therapeutic method for obtaining shiny hair characterized by applying a food supplement in form of two capsules once a day comprising
a. biotin in an amount of 0,05 to 2 mg based on each capsule, and
b. L-cystine in an amount of 1 to 5 mg based on each capsule, and
c. millet extract in an amount of 100 to 500 mg based on each capsule, and
d. pantothenic acid and/or a salt thereof in an amount of 5 to 50 mg based on each capsule, and
e. optionally at least one auxiliary ingredient
on a daily basis for at least eight weeks.

All percentages, ratios and proportions herein are by weight, unless otherwise specified. All temperatures are in degrees Celsius (°C) unless otherwise specified. If any unit or condition is not explicitly defined in the context of the present application a person skilled in the art will always consider the most obvious unit or condition. If in the context of the application any unit or condition is missing and this unit or condition is defined in another section of the application the same unit or condition applies where it is missing.

As used herein a food supplement means a concentrated source of nutrients or other substances with a nutritional or physiological effect which shall support people who suffer e.g. from specific deficiencies like scurvy. Such food supplements are usually marketed in "dose" form, such as pills, tablets, capsules, liquids in measured doses etc.

As used herein the hair appearance parameters i.e. shininess, softness, elasticity, smoothness, moisture and vitality mean what a professional hair dresser with several years of working experience understands under these expressions. Based on his professional experience the professional hair dresser can easily identify normal hair which is e.g. neither especially dull, nor especially shiny and for which he will give an average rating of 5.2 to 5.8 on a scale out of 1 to 10 if he assesses the same hair five times and calculates the mean value. Thus, it is important that the professional hair dressers have several years of working experience to have established a standard for calibrating what normal hair as described above means. There are also other methodologies available to assess the hair appearance parameters which seem to be more objective in the first place, but it is well known in the art that a professional hair dresser with several years of professional experience is more precise in his assessment than e.g. an analytical machine which is also reflected in the fact that well known and established test institutes use the same methodology as described above and in the experimental section below.

### Capsules

Capsules are widely used in the pharmaceutical field as oral dosage form containers for administration to humans and animals of, e.g. pharmaceuticals, veterinary products, food and dietary supplements. Advantages of capsules over other dosage forms may include better patient compliance, greater flexibility in dosage form design, and less expensive manufacturing processes. Pharmaceutical capsules are conventionally divided into soft shell capsules (hereinafter softgel capsules) and hard shell capsules (hereinafter hard capsules). The characteristics of softgel and hard capsules are well known in the pharmaceutical field.

Hard capsule shells are generally manufactured using dip molding processes involving the use of pins dipped into solutions of the different ingredients that are needed for the making of the capsule shell containers. Methods for the manufacturing of soft gelatin or softgel capsule shells are also known in the art and are different from hard capsule shell manufacturing. Manufacturing of soft gelatin or softgel capsule shells at a production scale was introduced by Robert Pauli Scherer in 1933 with the invention of a rotary die encapsulation machine. The rotary die process involves continuous formation of a heat seal between two ribbons of gelatin simultaneous with dosing of the fill liquid into each capsule. Although manufacturing process speed and efficiency has improved with time, the basic manufacturing principle remains essentially unchanged. Before the encapsulation process takes place, two sub-processes are often carried out simultaneously, yielding the two components of a softgel capsule: (a) the gel mass which will provide the softgel capsule shell, and (b) the fill matrix for the softgel capsule contents. Softgel capsules have a continuous gelatin or polysaccharide shell surrounding a liquid or gel like core, and are formed, filled and sealed in one operation.

Softgel capsule walls are typically thicker than two-piece hard gelatin capsules, and their walls comprise plasticizers such as, for example, glycerol, sorbitol and/or propylene glycol to make the shell elastic. Processes for making softgel capsule shells are known, and softgel capsules are available commercially. See, e.g. Aulton, Aulton's Pharmaceutics: The Design & Manufacture of Medicines, 527-533 (Kevin G Taylor, Ed., 3rd Ed., 2001). Softgel capsules have various advantages; they may show improved absorption of the filling, be easier to swallow, avoid dust handling issues, and have increased stability compared to other dosage forms. Softgel capsules may be filled with liquid fill such as oils and/or lipid soluble active ingredients such as pharmaceuticals, veterinary products, food and dietary supplements. In a preferred embodiment of the invention the capsules used in the present invention are softgel capsules. In another preferred embodiment the food supplement in the form of a capsule, preferably in the form of a softgel capsule is applied for at least 8 more preferably at least 10, and even more preferred at least 12 weeks.

### Biotin

Biotin was originally discovered in 1901 as part of essential factors for the growth of yeasts and first crystallized by F. Kögl in 1936. In the same time it was observed that toxic effects of overconsumption of raw eggs manifesting in skin lesions could be cured with treatment by a heat stable factor from yeast and liver, vitamin H, which turned to be out Biotin.

Biotin is obtained from the diet and bacterial synthesis in the intestine. Biotin is found in many foods but its concentration is very low. Milk, liver, kidney, egg yolk and some vegetable foods are good sources for human nutrition. The majority of biotin in meats and cereals appears to be protein bound. Dietary intake in Western populations is about 35-70 g/day. Only free biotin can be absorbed; bound biotin is released in the intestine by the enzyme biotinidase.

Biotin is important in the metabolism of all macronutrients via the four biotin-dependent carboxylase enzymes involved in fatty acid synthesis, gluconeogenesis and amino acid metabolism.

Reported findings of biotin deficiency in adults or children are quite similar to those documented originally when feeding egg white. In most patients thinning of hair, often with loss of hair color was reported. Also a skin rash, described as scaly (seborrheic) and red (eczematous) was present. Biotin deficiency may be caused by insufficient dietary uptake of biotin, drug-vitamin interactions and, perhaps, by increased biotin catabolism during pregnancy and in smokers.

In *in vitro* experiments with HeLa cells cultured in biotin-deficient medium a reduced rate of protein synthesis, DNA synthesis and growth was shown. On the other side, addition of exogenous biotin to the cells cultured in biotin-deficient medium, resulted in enhanced protein synthesis, DNA synthesis, and cell growth. In other experiments with a keratinocytes cell line (HaCaT) pharmacological biotin concentrations of 1 M, and 100 M caused a specific increase in cytokeratins which are normally induced upon terminal differentiation of epidermal cells *in vivo.*

In a study the effect of a daily oral dose of 2.5 mg biotin was studied in 93 patients with the symptoms hair-loss (mostly androgenetic alopecia) and reduced hair quality. The mean duration of treatment was 7.9±2.8 months. An obvious improvement of hair-loss was reported in 64% and a slight improvement in 9%. Brittle finger nails as an additional complaint were improved in 80%. If alopecia, decreased hair quality, and brittle finger nails occurred in combination improvement was observed frequently collectively. The study concluded to suggest biotin as an effective and well tolerated therapy in cases of alopecia.

Finally, the European Food Safety Authority EFSA published a scientific opinion on the substantiation of health claims related to biotin. The panel concluded that a cause and effect relationship has been established between the dietary intake of biotin and maintenance of normal hair. Further the panel stated that the evidence provided does not establish that inadequate intake of biotin leading to impaired functions occurs in the general EU population.

### Millet Extract

Millet extract contains fatty acids e.g. linoleic acid, alpha-linolenic acid and oleic acid. Millet extract is also a source of the phytosterol, miliacin, which is known to have anti-inflammatory properties and to help activate the repair processes of cells.

No studies on hair looked at millet extract treatment in isolation. However, a cell culture study demonstrated that miliacin significantly increased proliferation and metabolic activity in human keratinocytes. As a conclusion it was found that Miliacin appears to stimulate human keratinocytes indicating that it could promote hair growth.

### L-Cystine

L-cystine is the oxidized form of the amino acid L-cysteine that is a key hair component. L-cysteine is a sulphur-bearing amino acid that is essential for protein synthesis. L-cystine is a major constituent of keratin, the chief protein found in hair, nails and skin. Normal human hair has been shown to contain 14-16 % L-cystine by weight.

Two animal studies have demonstrated the prevention of experimental alopecia by dietary intake of L-cystine. Mice, treated with L-cystine and vitamin B6 prior to smoke exposure were less likely to suffer alopecia compared with un-supplemented mice. The relationship was dose-dependent.

One human study has investigated the role of L-cystine in hair growth. Forty healthy adults were randomly assigned to receive either a placebo capsule or a capsule containing gelatine and L-cystine for three months. Experimental sections of hair were examined at baseline and at three months. The results showed that hair growth and the diameter of hair fibres increased significantly in the active group only. Hair of treated adults was found to have higher sulphur content compared with baseline, suggesting that the L-cystine had been absorbed and incorporated into the hair. As a conclusion it was found that L-cystine is important for normal hair growth and quality. Animal research suggests that L-cystine may help prevent hair loss, while a controlled human trial demonstrated improvements in normal hair growth and quality following treatment with gelatine and L-cystine.

### Pantothenic Acid (Calcium Pantothenate)

Pantothenic acid is a precursor of co-enzyme A, which is important in the maintenance of rapidly proliferating cells, such as those found in hair. Experimental deficiency of pantothenic acid in animals causes pathogenic changes in the structure of hair and skin. In the art it was noted that the keratinic tiles on the hair of deficient animals were visibly looser and flakier than the tiles on hair of non-deficient animals (based on electron microscope methodology). This confirms that pantothenic acid is important for normal hair structure and quality. Pantothenic acid also stimulates metabolic activity in human keratinocytes, according to the previously mentioned cell culture study, which suggested that it might promote hair growth. Pantothenic acid is important in the nutritional support of normal hair growth and quality. It also appears to stimulate human keratinocytes suggesting that it could promote hair growth.

All the above mentioned active ingredients are useful and have proven their efficacy with regard to hair growth. In addition, the combination of all these actives i.e. biotin, L-cystine, millet extract and pantothenic acid and/or a salt thereof surprisingly supports the hair appearance and the effects of the present invention.

A second aspect of the invention is the non-therapeutic method for obtaining shiny hair, wherein a food supplement in form of two capsules comprising
a. biotin in an amount of 0,05 to 2 mg based on each capsule, and
b. L-cystine in an amount of 1 to 5 mg based on each capsule, and
c. millet extract in an amount of 100 to 500 mg based on each capsule, and
d. pantothenic acid and/or a salt thereof in an amount of 5 to 50 mg based on each capsule, and
e. optionally at least one auxiliary ingredient
is applied once a day on a daily basis for at least eight weeks.

For a person skilled in the art it is clear that all preferred embodiments of the first aspect of the invention are also preferred embodiments for the second aspect of the invention. Thus, also in the non-therapeutic method the capsules may be softgel capsulesand/or applied for at least 10 and more preferably for at least 12 weeks.

The compositions are of course not limited to the active ingredients as described above, but can comprise fillers, dyes, solvents, gelling agents, emulsifiers, stabilizers, flavoring agents, lubricants, humectants, thickeners or any other auxiliary useful for the present invention.

### Experimental Section

For the experiments 112 healthy male or female subjects (FAS: Full Analysis Set) were selected who were all in the age of 18 to 45 years with a mean age of 32±1 years and of Caucasian or French origin. Some of the subjects have been excluded during the study because of being not compliant with the study regulations so that a smaller group which was defined as the Per Protocol Population (PP) resulted. All subjects had a minimum hair length of 6 cm and agreed to keep this hair length throughout the study duration. All subjects confirmed to suffer at least occasionally from a mild seasonal hair loss, in particular in spring or autumn and all subjects were of skin type I to IV according to Fitzpatrick.

The Fitzpatrick skin type test is a numerical classification scheme for human skin color. It was developed in 1975 by Thomas B. Fitzpatrick as a way to estimate the response of different types of skin to UV light. It is based on the patient's reports of how their skin responds to the sun. The Fitzpatrick scale is a recognized tool for dermatological research into human skin pigmentation. The following list shows the six categories of the Fitzpatrick scale:
**Type I:** always burns, never tans - pale white skin, blond or red hair, blue eyes, freckles
**Type II:** usually burns, tans minimally - white skin, blond or red hair, blue, green or hazel eyes
**Type III:** sometimes mild burns, tans uniformly - cream white skin, fair with any hair or eye color
**Type IV:** burns minimally, always tans well - moderate brown skin
**Type V:** very rarely burns, tans very easily - dark brown skin
**Type VI:** never burns, never tans - deeply pigmented dark brown skin

All subjects participating in the experimental study agreed not to dye or change their hair color within three weeks prior to the start of the study and during the study, and all subjects had to avoid excessive UV exposure. The experimental study was conducted over a period of 86 days in which the subjects took two capsules (see Table 1) per day via oral administration.

**Table 1: Soft-gel capsule composition of the food supplement of the present invention.**

| **Name of Ingredients** | **Amount** / **wt %** |
|---|---|
| oily millet extract | 0.22 |
| calcium pantothenate | 1.88 |
| cystine | 0.32 |
| biotin | 0.01 |
| additives (wheat germ oil, beeswax, MgO) | 64.47 |
| shell of soft gel capsule (gelatine, glycerol, brown/red/yellow iron oxide, TiO₂, vanillin, p-methoxyacetophenone) | 33.10 |

On day 2, day 28, day 56 and day 84 of the 86 days period of the experimental study, three professional hair dresser having several years of professional experience assessed the overall hair appearance and beauty using five different beauty parameters, each one assessed by means of a 10-points-scale. The assessment was conducted based on the professional experience of the hair dressers and the below given scaling. A high rating close to 10 is a (very) good result and a low rating close to 1 is a significantly less good result.

The hair dressers used the following parameters and scale:
• Shining hair

| 1 | 2 | 3 | 4 | 5 | 6 | 7 | 8 | 9 | 10 |
|---|---|---|---|---|---|---|---|---|---|
| | | | | | | | | | |
| Dull hair | | | | | | | | Shiny hair | |

• Softness, elasticity of the hair

| 1 | 2 | 3 | 4 | 5 | 6 | 7 | 8 | 9 | 10 |
|---|---|---|---|---|---|---|---|---|---|
| | | | | | | | | | |
| Rough & stiff hair | | | | | | | | Soft & elastic or pliant hair | |

• Smoothness

| 1 | 2 | 3 | | | 4 5 6 7 8 9 | | | | 10 |
|---|---|---|---|---|---|---|---|---|---|
| | | | | | | | | | |
| Frizzy, «fried looking» hair | | | | | | | | Smooth, healthy looking hair | |

• Hair moisture

| 1 | 2 | 3 | 4 | 5 | 6 | 7 | 8 | 9 | 10 |
|---|---|---|---|---|---|---|---|---|---|
| | | | | | | | | | |
| Dry hair | | | | | | | | Moisturized hair | |

• Hair vitality

| 1 | 2 | 3 | 4 | 5 | 6 | 7 | 8 | 9 | 10 |
|---|---|---|---|---|---|---|---|---|---|
| | | | | | | | | | |
| Limp and lifeless hair | | | | | | | | Healthy hair & full of vitality | |

• Hair volume

| 1 | 2 | 3 | 4 | 5 | 6 | 7 | 8 | 9 | 10 |
|---|---|---|---|---|---|---|---|---|---|
| | | | | | | | | | |
| Lack of volume | | | | | | | | Lot of volume | |

### Results

By using the above scales, an individual score was calculated for each parameter and an inferential analysis was conducted to determine if the achieved results are statistically significant or not.

### Shining Hair Score

The table below shows the descriptive statistics of shining hair score for each time point and by population.

**Table 2: Descriptive analysis of shining hair score by population.**

| **Shining hair score** | | | |
|---|---|---|---|
| | | **Population** | |
| | | **FAS/Safety** | **PP** |
| **W0** | *N (miss)* | 112 (0) | 102 (0) |
| | *mean (SD)* | 4.41 (1.35) | 4.41 (1.32) |
| | *min ; max* | 1.00 ; 7.00 | 1.00 ; 7.00 |
| **W4** | *N (miss)* | 112 (0) | 102 (0) |
| | *mean (SD)* | 4.54 (1.41) | 4.49 (1.43) |
| | *min ; max* | 1.00 ; 9.00 | 1.00 ; 9.00 |
| **W8** | *N (miss)* | 110 (2) | 102 (0) |
| | *mean (SD)* | 4.98 (1.24) | 5.01 (1.22) |
| | *min; max* | 2.00 ; 8.00 | 2.00 ; 8.00 |
| **W12** | *N (miss)* | 109 (3) | 101 (1) |
| | *mean (SD)* | 6.09 (1.64) | 6.00 (1.56) |
| | *min ; max* | 3.00 ; 10.00 | 3.00 ; 9.00 |
| | | | |
| FAS: Full Analysis Set; PP: Per Protocol; W0: week 0, W4: week 4; W8: week 8, W12: week 12; N: number of subjects; mean: mean value: SD: standard deviation | | | |

The table below summarizes the change from baseline (week 0 = W0) for each time point (Wi) and by population of the shining hair score.

**Table 3: Inferential analysis of the shining hair score by population.**

| **Shining hair score** | | | | |
|---|---|---|---|---|
| | | **Population** | | |
| | | **FAS/Safety*** | **PP*** | **Sensitivity analysis**** |
| **W4-W0** | *Estimate* | 0.13 | 0.08 | 0.08 |
| | *SE* | 0.12 | 0.12 | 0.12 |
| | *p-value* | **0.3094** | **0.5271** | **0.5271** |
| **W8-W0** | *Estimate* | 0.58 | 0.60 | 0.60 |
| | *SE* | 0.14 | 0.13 | 0.13 |
| | *p-value* | **<0.0001** | **<0.0001** | **<0.0001** |
| **W12-W0** | *Estimate* | 1.68 | 1.59 | 1.59 |
| | *SE* | 0.21 | 0.22 | 0.22 |
| | *p-value* | **<0.0001** | **<0.0001** | **<0.0001** |
| | | | | |
| *values obtained from mixed ANOVA model for repeated measurements | | | | |
| **sensitivity analysis: Per Protocol population analyzed without outliers (none) | | | | |
| FAS: Full Analysis Set; PP: Per Protocol; W0: week 0, W4: week 4; W8: week 8, W12: week 12; SE: standard error; p-value: p-values lower than 0.001 show that the parameter effects are significant | | | | |

For the FAS population, a significant improvement in hair shining score is observed from W8:
- Of +13% at W8 (+0.6 point on the 10 points scale);
- Of +38% at W12 (+1.7 point on the 10 points scale).

For the PP population, a significant improvement is also observed from W8.

### Softness, elasticity of the hair

The table below shows the descriptive statistics of softness, elasticity of hair score for each time point and by population.

**Table 4: Descriptive analysis of softness, elasticity of the hair score by population.**

| **Softness, elasticity of the hair score** | | | |
|---|---|---|---|
| | | **Population** | |
| | | **FAS/Safety** | **PP** |
| **W0** | *N (miss)* | 112 (0) | 102 (0) |
| | ***mean (SD)*** | **4.45 (1.27)** | **4.43 (1.24)** |
| | *min ; max* | 2.00 ; 8.00 | 2.00 ; 8.00 |
| **W4** | *N (miss)* | 112 (0) | 102 (0) |
| | ***mean (SD)*** | **4.62 (1.37)** | **4.57 (1.38)** |
| | *min ; max* | 1.00 ; 9.00 | 1.00 ; 9.00 |
| **W8** | *N (miss)* | 110 (2) | 102 (0) |
| | ***mean (SD)*** | **5.05 (1.18)** | **5.03 (1.14)** |
| | *min ; max* | 2.00 ; 8.00 | 2.00 ; 8.00 |
| **W12** | *N (miss)* | 109 (3) | 101 (1) |
| | ***mean (SD)*** | **5.98 (1.64)** | **5.88 (1.58)** |
| | *min ; max* | 3.00; 10.00 | 3.00 ; 9.00 |
| | | | |
| FAS: Full Analysis Set; PP: Per Protocol; W0: week 0, W4: week 4; W8: week 8, W12: week 12; N: number of subjects; mean: mean value: SD: standard deviation | | | |

The table below summarizes the change from baseline (W0) for each time point (Wi) and by population of the softness and elasticity of the hair score.

**Table 5: Inferential analysis of the softness, elasticity of the hair score by population.**

| **Softness, elasticity of the hair score** | | | | |
|---|---|---|---|---|
| | | **Population** | | |
| | | **FAS/Safety*** | **PP*** | **Sensitivity analysis**** |
| **W4-W0** | *Estimate* | 0.17 | 0.14 | 0.30 |
| | *SE* | 0.12 | 0.12 | 0.11 |
| | *p-value* | **0.1494** | **0.2613** | **0.0079** |
| **W8-W0** | *Estimate* | 0.62 | 0.60 | 0.60 |
| | *SE* | 0.13 | 0.14 | 0.14 |
| | *p-value* | **<0.0001** | **<0.0001** | **<0.0001** |
| **W12-W0** | *Estimate* | 1.53 | 1.45 | 1.45 |
| | *SE* | 0.21 | 0.21 | 0.21 |
| | *p-value* | **<0.0001** | **<0.0001** | **<0.0001** |
| | | | | |
| *values obtained from mixed ANOVA model for repeated measurements | | | | |
| **sensitivity analysis: Per Protocol population analyzed without outliers | | | | |
| FAS: Full Analysis Set; PP: Per Protocol; W0: week 0, W4: week 4; W8: week 8, W12: week 12; SE: standard error; p-value: p-values lower than 0.001 show that the parameter effects are significant | | | | |

For the FAS population, a significant improvement in hair softness and elasticity score is observed from W8:
- Of +13% at W8 (+0.6 point on the 10 points scale);
- Of+34% at W12 (+1.5 point on the 10 points scale).

For the PP population, an improvement in hair softness and elasticity is also observed from W8.

### Smoothness score

The table below shows the descriptive statistics of smoothness score for each time point and by population.

**Table 6: Descriptive analysis of smoothness score by population.**

| **Smoothness score** | | | |
|---|---|---|---|
| | | **Population** | |
| | | **FAS/Safety** | **PP** |
| **W0** | *N (miss)* | 112 (0) | 102 (0) |
| | ***mean (SD)*** | **4.22 (1.23)** | **4.21 (1.18)** |
| | *min ; max* | 1.00 ; 8.00 | 1.00 ; 6.00 |
| **W4** | *N (miss)* | 112 (0) | 102 (0) |
| | ***mean (SD)*** | **4.49 (1.58)** | **4.42 (1.56)** |
| | *min ; max* | 1.00 ; 10.00 | 1.00 ; 10.00 |
| **W8** | *N (miss)* | 110 (2) | 102 (0) |
| | ***mean (SD)*** | **5.05 (1.33)** | **5.02 (1.23)** |
| | *min ; max* | 2.00 ; 9.00 | 2.00 ; 9.00 |
| **W12** | *N (miss)* | 109 (3) | 101 (1) |
| | ***mean (SD)*** | **5.70 (1.77)** | **5.60 (1.72)** |
| | *min; max* | 2.00 ; 10.00 | 2.00 ; 10.00 |
| | | | |
| FAS: Full Analysis Set; PP: Per Protocol; W0: week 0, W4: week 4; W8: week 8, W12: week 12; N: number of subjects; mean: mean value: SD: standard deviation | | | |

The table below summarizes the change from baseline (W0) for each time point (Wi) and by population of the smoothness score.

**Table 7: Inferential analysis of the smoothness score by population.**

| **Smoothness score** | | | | |
|---|---|---|---|---|
| | | **Population** | | |
| | | **FAS/Safety*** | **PP*** | **Sensitivity analysis**** |
| **W4-W0** | *Estimate* | 0.27 | 0.22 | 0.27 |
| | *SE* | 0.14 | 0.14 | 0.09 |
| | *p-value* | **0.0554** | **0.1315** | **0.0047** |
| **W8-W0** | *Estimate* | 0.84 | 0.81 | 0.81 |
| | *SE* | 0.14 | 0.15 | 0.15 |
| | *p-value* | **<0.0001** | **<0.0001** | **<0.0001** |
| **W12-W0** | *Estimate* | 1.47 | 1.40 | 1.34 |
| | *SE* | 0.21 | 0.22 | 0.21 |
| | *p-value* | **<0.0001** | **<0.0001** | **<0.0001** |
| *values obtained from mixed ANOVA model for repeated measurements | | | | |
| **sensitivity analysis: Per Protocol population analyzed without outliers | | | | |
| FAS: Full Analysis Set; PP: Per Protocol; W0: week 0, W4: week 4; W8: week 8, W12: week 12; SE: standard error; p-value: p-values lower than 0.001 show that the parameter effects are significant | | | | |

For the FAS population, a significant improvement in hair smoothness score is observed from W8:
- Of +20% at W8 (+0.8 point on the 10 points scale);
- Of +35% at W12 (+1.5 point on the 10 points scale).

For the PP population, a similar improvement in hair smoothness is observed from W8.

### Hair moisture score

The table below shows the descriptive statistics of hair moisture score for each time point and by population.

**Table 8: Descriptive analysis of hair moisture score by population.**

| **Hair moisture score** | | | |
|---|---|---|---|
| | | **Population** | |
| | | **FAS/Safety** | **PP** |
| **W0** | *N (miss)* | 112 (0) | 102 (0) |
| | ***mean (SD)*** | **4.24 (1.47)** | **4.21 (1.42)** |
| | *min ; max* | 1.00 ; 8.00 | 1.00 ; 6.00 |
| **W4** | *N (miss)* | 112 (0) | 102 (0) |
| | ***mean (SD)*** | **4.69 (1.70)** | **4.65 (1.75)** |
| | *min ; max* | 1.00 ; 10.00 | 1.00 ; 10.00 |
| **W8** | *N (miss)* | 110 (2) | 102 (0) |
| | ***mean (SD)*** | **5.26 (1.59)** | **5.28 (1.57)** |
| | *min ; max* | 1.00 ; 9.00 | 1.00 ; 9.00 |
| **W12** | *N (miss)* | 109 (3) | 101 (1) |
| | ***mean (SD)*** | **6.18 (1.78)** | **6.11 (1.73)** |
| | *min ; max* | 1.00 ; 10.00 | 1.00 ; 9.00 |
| | | | |
| FAS: Full Analysis Set; PP: Per Protocol; W0: week 0, W4: week 4; W8: week 8, W12: week 12; N: number of subjects; mean: mean value: SD: standard deviation | | | |

The table below summarizes the change from baseline (W0) for each time point (Wi) and by population of the hair moisture score.

**Table 9: Inferential analysis of the hair moisture score by population.**

| **Hair moisture score** | | | | |
|---|---|---|---|---|
| | | **Population** | | |
| | | **FAS/Safety*** | **PP*** | **Sensitivity analysis**** |
| **W4-W0** | *Estimate* | 0.45 | 0.44 | 0.44 |
| | *SE* | 0.15 | 0.16 | 0.16 |
| | *p-value* | **0.0039** | **0.0065** | **0.0065** |
| **W8-W0** | *Estimate* | 1.04 | 1.08 | 1.08 |
| | *SE* | 0.15 | 0.15 | 0.15 |
| | *p-value* | **<0.0001** | **<0.0001** | **<0.0001** |
| **W12-W0** | *Estimate* | 1.96 | 1.91 | 1.94 |
| | *SE* | 0.19 | 0.20 | 0.20 |
| | *p-value* | **<0.0001** | **<0.0001** | **<0.0001** |
| | | | | |
| *values obtained from mixed ANOVA model for repeated measurements | | | | |
| **sensitivity analysis: Per Protocol population analyzed without outliers | | | | |
| FAS: Full Analysis Set; PP: Per Protocol; W0: week 0, W4: week 4; W8: week 8, W12: week 12; SE: standard error; p-value: p-values lower than 0.001 show that the parameter effects are significant | | | | |

For the FAS population, a significant improvement in hair moisture score is observed from W4:
- Of +11% at W4 (+0.5 point on the 10 points scale);
- Of +24% at W8 (+1.0 point on the 10 points scale);
- Of +46% at W12 (+1.9 point on the 10 points scale).

For the PP population, a similar improvement is observed.

### Hair vitality score

The table below shows the descriptive statistics of hair vitality score for each time point and by population.

**Table 10: Descriptive analysis of hair vitality score by population.**

| **Hair vitality score** | | | |
|---|---|---|---|
| | | **Population** | |
| | | **FAS/Safety** | **PP** |
| **W0** | *N (miss)* | 112 (0) | 102 (0) |
| | ***mean (SD)*** | **4.23 (1.45)** | **4.18 (1.40)** |
| | *min ; max* | 1.00 ; 8.00 | 1.00 ; 6.00 |
| **W4** | *N (miss)* | 112 (0) | 102 (0) |
| | ***mean (SD)*** | **4.71 (1.66)** | **4.65 (1.67)** |
| | *min ; max* | 1.00 ; 9.00 | 1.00 ; 9.00 |
| **W8** | *N (miss)* | 110 (2) | 102 (0) |
| | ***mean (SD)*** | **5.35 (1.61)** | **5.36 (1.58)** |
| | *min ; max* | 1.00 ; 10.00 | 1.00 ; 10.00 |
| **W12** | *N (miss)* | 109 (3) | 101 (1) |
| | ***mean (SD)*** | **6.24 (1.80)** | **6.15 (1.76)** |
| | *min; max* | 1.00 ; 10.00 | 1.00 ; 10.00 |
| | | | |
| FAS: Full Analysis Set; PP: Per Protocol; W0: week 0, W4: week 4; W8: week 8, W12: week 12; N: number of subjects; mean: mean value: SD: standard deviation | | | |

The table below summarizes the change from baseline (W0) for each time point (Wi) and by population of the hair vitality score.

**Table 11: Inferential analysis of the hair vitality score by population.**

| **Hair vitality score** | | | | |
|---|---|---|---|---|
| | | **Population** | | |
| | | **FAS/Safety*** | **PP*** | **Sensitivity analysis**** |
| **W4-W0** | *Estimate* | 0.48 | 0.47 | 0.47 |
| | *SE* | 0.15 | 0.16 | 0.16 |
| | *p-value* | **0.0015** | **0.0032** | **0.0032** |
| **W8-W0** | *Estimate* | 1.13 | 1.19 | 1.15 |
| | *SE* | 0.14 | 0.14 | 0.14 |
| | *p-value* | **<0.0001** | **<0.0001** | **<0.0001** |
| **W12-W0** | *Estimate* | 2.03 | 1.98 | 2.01 |
| | *SE* | 0.19 | 0.20 | 0.20 |
| | *p-value* | **<0.0001** | **<0.0001** | **<0.0001** |
| | | | | |
| *values obtained from mixed ANOVA model for repeated measurements | | | | |
| **sensitivity analysis: Per Protocol population analyzed without outliers | | | | |
| FAS: Full Analysis Set; PP: Per Protocol; W0: week 0, W4: week 4; W8: week 8, W12: week 12; SE: standard error; p-value: p-values lower than 0.001 show that the parameter effects are significant | | | | |

For the FAS population, a significant improvement in hair vitality score is observed from W4:
- Of +11% at W4 (+0.5 point on the 10 points scale);
- Of +26% at W8 (+1.1 point on the 10 points scale);
- Of +48% at W12 (+2.0 points on the 10 points scale).

For the PP population, an increase in hair vitality score is also observed.

### Hair volume score

The table below shows the descriptive statistics of hair volume score for each time point and by population.

**Table 12: Descriptive analysis of hair volume score by population.**

| **Hair volume score** | | | |
|---|---|---|---|
| | | **Population** | |
| | | **FAS/Safety** | **PP** |
| **W0** | *N (miss)* | 112 (0) | 102 (0) |
| | ***mean (SD)*** | **5.29 (1.76)** | **5.20 (1.76)** |
| | *min ; max* | 1.00 ; 10.00 | 1.00 ; 10.00 |
| **W4** | *N (miss)* | 112 (0) | 102 (0) |
| | ***mean (SD)*** | **5.28 (1.50)** | **5.22 (1.51)** |
| | *min ; max* | 1.00 ; 9.00 | 1.00 ; 9.00 |
| **W8** | *N (miss)* | 110 (2) | 102 (0) |
| | ***mean (SD)*** | **5.32 (1.51)** | **5.24 (1.45)** |
| | *min ; max* | 1.00 ; 10.00 | 1.00 ; 10.00 |
| **W12** | *N (miss)* | 109 (3) | 101 (1) |
| | ***mean (SD)*** | **6.20 (1.80)** | **6.09 (1.73)** |
| | *min; max* | 3.00; 10.00 | 3.00; 10.00 |
| | | | |
| FAS: Full Analysis Set; PP: Per Protocol; W0: week 0, W4: week 4; W8: week 8, W12: week 12; N: number of subjects; mean: mean value: SD: standard deviation | | | |

The table below summarizes the change from baseline (W0) for each time point (Wi) and by population of the hair volume score.

**Table 13: Inferential analysis of the hair volume score by population.**

| **Hair volume score** | | | | |
|---|---|---|---|---|
| | | **Population** | | |
| | | **FAS/Safety*** | **PP*** | **Sensitivity analysis**** |
| **W4-W0** | *Estimate* | -0.02 | 0.02 | 0.04 |
| | *SE* | 0.15 | 0.14 | 0.15 |
| | *p-value* | **0.9023** | **0.8925** | **0.7899** |
| **W8-W0** | *Estimate* | 0.03 | 0.04 | -0.01 |
| | *SE* | 0.17 | 0.17 | 0.17 |
| | *p-value* | **0.8596** | **0.8169** | **0.9586** |
| **W12-W0** | *Estimate* | 0.92 | 0.90 | 0.92 |
| | *SE* | 0.19 | 0.19 | 0.20 |
| | *p-value* | **<.0001** | **<.0001** | **<.0001** |
| *values obtained from mixed ANOVA model for repeated measurements | | | | |
| **sensitivity analysis: Per Protocol population analyzed without outliers | | | | |
| FAS: Full Analysis Set; PP: Per Protocol; W0: week 0, W4: week 4; W8: week 8, W12: week 12; SE: standard error; p-value: p-values lower than 0.001 show that the parameter effects are significant | | | | |

For the FAS population, a slight significant improvement in hair volume score of+17% (0.9 point on the 10 points scale) is observed at W12. For the PP population, similar results are observed.

## Claims

1. Use of a food supplement in the form of a capsule, wherein the food supplement comprises
a. Biotin in an amount of 0,05 to 2 mg based on each capsule, and
b. L-cystine in an amount of 1 to 5 mg based on each capsule, and
c. millet extract in an amount of 100 to 500 mg based on each capsule, and
d. pantothenic acid and/or a salt thereof in an amount of 5 to 50 mg based on each capsule, and
e. optionally at least one auxiliary ingredient
for obtaining shiny hair or soft and elastic hair or smooth hair moisturized hair or vital hair when applied in the form of two capsules once a day on a daily basis for at least eight weeks.

2. Use of a food supplement according to claim 1, **characterized by** that the capsule is a soft-gel capsule.

3. Use of a food supplement according to any of the claims 1 to 2, **characterized by** that the food supplement is applied for at least 10, more preferably at least 12 weeks.

4. Non-therapeutic method for obtaining shiny hair **characterized by** applying a food supplement in form of two capsules once a day comprising
a. Biotin in an amount of 0,05 to 2 mg based on each capsule, and
b. L-cystine in an amount of 1 to 5 mg based on each capsule, and
c. millet extract in an amount of 100 to 500 mg based on each capsule, and
d. pantothenic acid and/or a salt thereof in an amount of 5 to 50 mg based on each capsule, and
e. optionally at least one auxiliary ingredient
on a daily basis for at least eight weeks.

5. Non-therapeutic method according to claim 4 **characterized by** that the capsule is a soft-gel capsule.

6. Non-therapeutic method according to any of claims 4 to 5 **characterized by** that the food supplement is applied for at least 10, more preferably at least 12 weeks.

## Patentansprüche

1. Verwendung eines Nahrungsergänzungsmittels in Form einer Kapsel, wobei das Nahrungsergänzungsmittel
a. Biotin in einer Menge von 0,05 bis 2 mg, bezogen auf jede Kapsel, und
b. L-Cystin in einer Menge von 1 bis 5 mg, bezogen auf jede Kapsel, und
c. Hirseextrakt in einer Menge von 100 bis 500 mg, bezogen auf jede Kapsel, und
d. Pantothensäure und/oder ein Salz davon in einer Menge von 5 bis 50 mg, bezogen auf jede Kapsel, und
e. gegebenenfalls mindestens einen Hilfsbestandteil umfasst, zum Erhalt von glänzendem Haar oder weichem und elastischem Haar oder glattem Haar, befeuchtetem Haar oder vitalem Haar bei Anwendung in Form von zwei Kapseln einmal pro Tag auf täglicher Basis über einen Zeitraum von mindestens acht Wochen.

2. Verwendung eines Nahrungsergänzungsmittels nach Anspruch 1, **dadurch gekennzeichnet, dass** es sich bei der Kapsel um eine Weichgelkapsel handelt.

3. Verwendung eines Nahrungsergänzungsmittels nach einem der Ansprüche 1 bis 2, **dadurch gekennzeichnet, dass** das Nahrungsergänzungsmittel über einen Zeitraum von mindestens 10 Wochen, weiter bevorzugt mindestens 12 Wochen, angewendet wird.

4. Nichttherapeutisches Verfahren zum Erhalt von glänzendem Haar, **gekennzeichnet durch** das Anwenden eines Nahrungsergänzungsmittels in Form von zwei Kapseln einmal pro Tag, umfassend
a. Biotin in einer Menge von 0,05 bis 2 mg, bezogen auf jede Kapsel, und
b. L-Cystin in einer Menge von 1 bis 5 mg, bezogen auf jede Kapsel, und
c. Hirseextrakt in einer Menge von 100 bis 500 mg, bezogen auf jede Kapsel, und
d. Pantothensäure und/oder ein Salz davon in einer Menge von 5 bis 50 mg, bezogen auf jede Kapsel, und
e. gegebenenfalls mindestens einen Hilfsbestandteil, auf täglicher Basis über einen Zeitraum von mindestens acht Wochen.

5. Nichttherapeutisches Verfahren nach Anspruch 4, **dadurch gekennzeichnet, dass** es sich bei der Kapsel um eine Weichgelkapseln handelt.

6. Nichttherapeutisches Verfahren nach einem der Ansprüche 4 bis 5, **dadurch gekennzeichnet, dass** das Nahrungsergänzungsmittel über einen Zeitraum von mindestens 10 Wochen, weiter bevorzugt mindestens 12 Wochen, angewendet wird.

## Revendications

1. Utilisation d'un supplément alimentaire sous forme d'une capsule, le supplément alimentaire comprenant
a. de la biotine en une quantité de 0,05 à 2 mg sur la base de chaque capsule, et
b. de la L-cystine en une quantité de 1 à 5 mg sur la base de chaque capsule, et
c. un extrait de millet en une quantité de 100 à 500 mg sur la base de chaque capsule, et
d. de l'acide pantothénique et/ou un sel correspondant en une quantité de 5 à 50 mg sur la base de chaque capsule, et
e. éventuellement au moins un ingrédient auxiliaire
pour l'obtention de cheveux brillants ou de cheveux doux et souples ou de cheveux lisses, de cheveux hydratés ou de cheveux vitaux lorsqu'appliqué sous forme de deux capsules une fois par jour sur une base quotidienne pendant au moins huit semaines.

2. Utilisation d'un supplément alimentaire selon la revendication 1, **caractérisée en ce que** la capsule est une capsule de gel souple.

3. Utilisation d'un supplément alimentaire selon l'une quelconque des revendications 1 et 2, **caractérisée en ce que** le supplément alimentaire est appliqué pendant au moins 10, plus préférablement au moins 12 semaines.

4. Procédé non thérapeutique pour l'obtention de cheveux brillants **caractérisé par** l'application d'un supplément alimentaire sous forme de deux capsules une fois par jour comprenant
a. de la biotine en une quantité de 0,05 à 2 mg sur la base de chaque capsule, et
b. de la L-cystine en une quantité de 1 à 5 mg sur la base de chaque capsule, et
c. un extrait de millet en une quantité de 100 à 500 mg sur la base de chaque capsule, et
d. de l'acide pantothénique et/ou un sel correspondant en une quantité de 5 à 50 mg sur la base de chaque capsule, et
e. éventuellement au moins un ingrédient auxiliaire
sur une base quotidienne pendant au moins huit semaines.

5. Procédé non thérapeutique selon la revendication 4 **caractérisé en ce que** la capsule est une capsule de gel souple.

6. Procédé non thérapeutique selon l'une quelconque des revendications 4 et 5 **caractérisé en ce que** le supplément alimentaire est appliqué pendant au moins 10, plus préférablement au moins 12 semaines.
